Europäisches Patentamt

**European Patent Office**

**Office européen des brevets**

(11) Publication number: **0 318 156**
**A2**

# (12) EUROPEAN PATENT APPLICATION

(21) Application number: **88310131.3**

(22) Date of filing: **27.10.88**

(51) Int. Cl.⁴: **B65F 1/00 , A61M 5/32**

(30) Priority: **28.10.87 GB 8725246**

(43) Date of publication of application:
**31.05.89 Bulletin 89/22**

(84) Designated Contracting States:
**AT BE CH DE ES FR GB GR IT LI LU NL SE**

(71) Applicant: **C.P.R. MEDICAL LIMITED**
**Britannic House Market Place**
**Sutton-in-Ashfield**
**Nottingham NG17 1DH(GB)**

(72) Inventor: **Newman Christopher Norman**
**13 Glebe Close**
**Holmewood, Chesterfield(GB)**

(74) Representative: **Lewis, Samuel Hewitt et al**
**FORRESTER & BOEHMERT**
**Widenmayerstrasse 4/I**
**D-8000 München 22(DE)**

(54) **Apparatus and method for the disposal of contaminated or dangerous articles.**

(57) An apparatus and method for the disposal of contaminated or dangerous articles such as needles (20), (called "sharps") which have been used for the giving of injections or taking of blood, the apparatus comprising a container (11), having a receiving means (14), the receiving means having a hollow (19) to receive the article (20) and a closure member (22) movable from a closed position in which the article is prevented from passing from the hollow into the container, to an open position in which the article is able to pass from the hollow into the container (11), operating means (27) which are operable externally of the container to move the closure member (22) from the closed position to the open position, resilient means (25) to return the closure member (22) to the closed position when the operating means are released.

FIG 1

## Apparatus and method for the disposal of contaminated or dangerous articles

Description of Invention

This invention relates to an apparatus and method for the disposal of contaminated or dangerous articles such as needles, (called "sharps") which have been used for the giving of injections or taking blood.

Previously it has been common medical practice to separate sharps from syringes after use and dispose of the used shaarps, in a container which has an opening through which the sharps are introduced into the container. Such containers are commonly made of cardboard to facilate incineration when full.

The syringes have been disposed of separately to the sharps, for example in a liquid impermeable bin to retain any liquid which may leak from the syringes.

Known used sharps containers suffer from the disadvantage that they can be overfilled thereby presenting a risk to any person placing a further used sharp into the container.

Furthermore recently, it is has been medical practice not to separate the used sharps from their syringes prior to disposal because of the risk of injury this presents from the used sharp. It will be apparent that a used sharp could be contaminated with the blood of a patient suffering from an infectious disease such as Hepatitis or Aids, which disease could be transmitted in this way.

Acording to a first aspect of the invention we provide an apparatus for the disposal of a contaminated or dangerous article comprising a container, characterised in that the apparatus has a receiving means, the receiving means having a hollow to receive the article and a closure member movable from a closed position in which the article is prevented from passing from the hollow into the container, to an open position in which the article is able to pass from the hollow into the container, operating means which are operable externally of the container to move the closure member from the closed position to the open position, resilient means to return the closure member to the closed position when the operating means are released.

The apparatus in accordance with the first aspect of the invention provides significant advantages over prior arrangements.

First, once the article is recieved in the container, it is not possible easily to remove the article, thereby overcoming the risk of, for example a drug addict, stealing a used sharp from a container.

Secondly, an article such as a used sharp can be disposed of into the container simply by inserting the sharp into the hollow and then operating the operating means so that there is no risk of injury from any sharp already received in the container.

Preferably the closure member opens in the same general direction in which the article moves to pass from the hollow into the container. This provides a third advantage in that as the container is filled, eventually the closure member is prevented from being opened, by articles already in the container, ensuring that the container cannot be overfilled.

Preferably, the container is made from a liquid impermeable material such as plastic, so that in the event that liquid for example from used syringes, leaks into the container, the container does not become weakened.

It will be appreciated that this provides a significant fourth advantage over cardboard containers which can become sodden with the ensuring risk that used sharps will be able to pass through the container wall and cause injury.

According to a second aspect of the invention we provide a method of disposing of a contaminated or dangerous article using an apparatus in accordance with the first aspect of the invention, characterised in that the method comprises placing the article in the hollow to the receiving means, operating the operating means to cause the closure member to move to the open position and allow the article to pass into the container.

The invention will now be described with the aid of the accompanying drawings in which:

FIGURE 1 is a side illustrative perspective view of an apparatus in accordance with the invention.

FIGURE 2 is an illustrative plan view of part of the apparatus of Figure 1.

FIGURE 3 is an end view of the part of the apparatus of Figure 2 taken on arrow A.

Referring to the drawings, an apparatus 10 for used sharps comprises a container 11 which in the example shown is of generally cylindrical configuration, but could be of another configuration as required.

In this embodiment, the container 11 has a lid L engaged with a body B in such a way e.g. snap interfit, that once engaged it is difficult or impossible to remove the lid L from the body B.

The container 11 is made predominently in a plastic material, for example by moulding and is thus impermeable to the penetration of liquids.

The container 11 has an opening 12 in the lid L thereof, the opening 12 receiving a receiving means 14 which is positioned therein.

The receiving means 14 comprises a tube of generally rectangular cross section, although a tube of other cross section may be provided as required, each side of the rectangle having an outwardly extending flange 16a, 16b, 16c, 16d provided at one end of the tube 15. The flanges 16a - 16d are preferably adhered to the lid L but could alternatively have openings to receive fasteners such as rivets or screws, which engage with the lid L of the container 11 to secure the receiving means 14 in the position shown.

The tube 15 defines an internal hollow 19 into which articles, in the present example, used syringes and sharps, one of which is shown at 20 in the hollow 19, can be placed.

At the lower end of the tube 15, a closure member 22 is provided, the closure member 22 normally extending across the hollow 19 and preventing the syringe and sharp 20 from passing from the hollow 19 into the container 11.

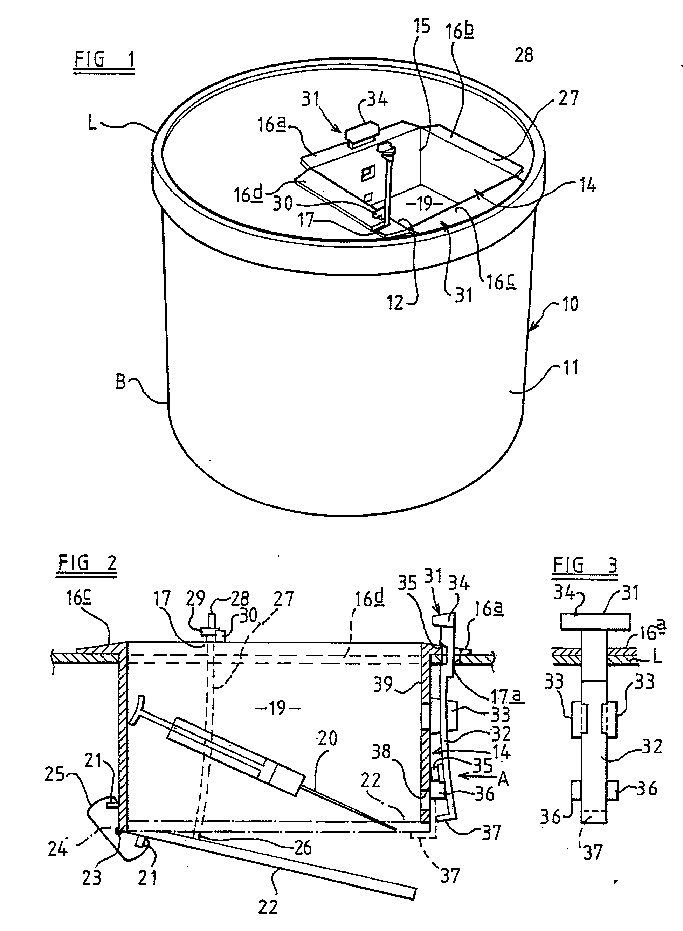

The closure member 22 is hinged to the tube wall via a hinge 23 which comprises a thin web of plastic material and thus the closure member 22 is pivotable about a hinge axis 24 from the closed position shown in dotted lines in figure 2, to the position shown in full lines.

A resilient menas comprising a "C" spring 25 acts between abutments 21 on the closure member 22 and adjacent side of the receiving means 14, to retain the closure member 22 in the closed position, but the closure member 22 can be moved against the force of the resilient means 25 by an operating means.

The operating means comprises a push rod 27 which is connected at a lower end 26 thereof to the closure member 22, the push rod 27 passing upwardly through an opening 17 in the flange 16d to a push button or other manually engageable member 28.

Preferably, the closure member 22 and push rod 27 are all formed integrally with the remainder of the receiving means 14, in a generally flat condition as a plastic moulding. The receiving means 14 is then formed by scoring and bending the moulding to the required configuration. Thus the opening 17 in flange 16d to receive the push rod 27 may comprise a slot so that the push rod 27 is retained on three sides by the slot, and on the fourth side by a peripheral part of the opening 12 in the lid L.

It will be appreciated that by pushing rod 27 downwardly using the button 28, from outside the container 11, the closure member 22 will be moved from the closed position (dotted lines) to an open position (full lines) by pivoting about the hinge axis 24 the connevtion between the rod 27 and member 22 permitting the lower end 26 of the rod 27 to pivot relative to the member 22. Thus any article 20 placed in the hollow 19 of the receiving means 14 will pass from the hollow 19 into the container 11.

Adjacent the button 28, a locking formation 29 is provided which may be engaged with a corresponding hook formation 30 provided on flange 16d when push rod 17 is depressed, and rotated, to maintain the closure member 22 in an open position if required.

As the container 11 is filled, it will be appreciated that the level of articles in the container will rise. Eventually, the articles will assume positions such that the closure member 22 cannot be opened by operating the operating means, because the closure member 22 moves in the same general direction that the articles must move to pass from the hollow 19 into the container.

Thus the container cannot be overfilled.

When the container is full, the push rod 27 may be removed by twisting to break the connection between the lower end 26 of rod 27 and closure member 22 so that it is then necessary for further syringes and sharps 20 to be disposed of using a fresh empty apparatus 10.

If desired, a locking means such as shown at 31 may be provided to lock the closure member 22 closed when the container is full.

The locking means 31 comprises an elongate slider 32 which is held in the position shown in Figures 2 and 3, by a pair of formations 33 between which the slider 32 slides. The slider extends upwardly through an opening 17 a in flange 16a (again a slot) to a manually engageable part 34 which has a step 35 which engages with flange 16a to prevent the slider 32 being accidentally moved downwards.

At its lower end, the slider 32 has a lug 35 which is received between a further, plain, pair of formations 36, and at its very lowest end, an inwardly extending flange 37 is provided.

When the container 11 is full, and the closure member 22 is closed, by depressing the part 34, at the same time disengaging the step 35 from flange 16a, the slider 32 can be moved downwards until the flange 37 engages beneath the closure member 22 as shown in dotted lines in figure 2 and the lug 35 engages in an opening 38 in the adjacent side wall 39 of the hollow 19. Thus the closure member 22 cannot then be opened, and the locking means 30 cannot be released from outside the container 11.

This is of course only one example of a locking means which may be provided, there being many alternative practical arrangements.

The closure member 22 is preferably made from a soft material so that in the event that in normal use anyone attempts to force a used sharp into the container 11 by pushing the closure member 22 open using the sharp, the sharp will simply dig in or pass through the closure member 22 without opening the closure member 22. Thus the risk of a sharp breaking is minimised, but due to the depth of the hollow 19, even in the event that a sharp 20 were to break in the hollow 19, the break should be contained so that no part of the broken sharps leaves the hollow to present any risk of injury.

Various modifications are possible without departing from the scope of the invention.

As shown, the container 11 is generally cylindrical having a lid L although the container could extend upwardly to a neck in which the opening 12 is provided. Alternatively, the receiving means 14 or at least the tube 15 thereof, could be provided integrally within the lid L or neck of the container.

The tube 15 could be of an alternative cross section to retangular as required e.g. circular, and can be fabricated and secured in the opening 12 in any manner desired.

In place of the spring 25 any other resilient means normally to urge the closure member 22 to the closed position could be provided, and the closure member 22 could in an alternative arrangement, be otherwise hinged relative to the tube 15 or be otherwise secured relative thereto and be openable from the closed to the open position. In place of the opening means comprising a rod 27 any other desired operating means operable from externally of the container, for moving the closure member 22 against the force of the resilient means 25 to allow articles to pass from the hollow 19 into the container, may be provided e.g. a pull cord may be provided.

As described, the receiving means 15 can be manufactured from a generally flat sheet or moulding of suitable material, scored and folded to the configuration shown, or injection moulded to shape.

If desired, instead of the entire receiving means being moulded separately from the lid L, the receiving means 14 may be moulded integrally with the lid L.

Where the receiving means is moulded, but separately from the lid, the flanges 16a-16d which overlie the lid L may be continuous to provide a seal between the receiving means and the lid rather than having four flaps 16a-16d as described.

As described, the container 11 comprises a lid L and a body B so that bodies B can be stacked to facilate storage, although if desired, the lid L and body B may be provided integrally.

The features disclosed in the foregoing description in the following claims or the accompanying drawings, expressed in their specific forms or in terms of a means for performing the disclosed function, or a method or process for attaining the disclosed result, or a class or group of substances or compositions, as appropriate, may, separately or in any combination of such features, be utilised for realising the invention in diverse forms thereof.

**Claims**

1. An apparatus (10) for the disposal of a contaminated or dangerous article (20) comprising a container (11), characterised in that the container has a receiving means (14), the receiving means having a hollow (19) to receive the article (20) and a closure member (22) movable from a closed position in which the article (20) is prevented from passing from the hollow (19) into the container (11), to an open position in which the article (20) is able to pass from the hollow (19) into the container (11), operating means (27) which are operable externally of the container (11) to move the closure member (23) from the closed position to the open position, resilient means (25) to return the closure member (22) to the closed position when the operating means (27) are released

2. An apparatus according to claim 1 characterised in that the closure member (22) opens in the same general direction in which the article (20) moves to pass from the hollow (19) into the container (11).

3. An apparatus according to claim 1 or claim 2 characterised in that means (31) are provided to lock the closure member (22) in a closed position.

4. An apparatus according to any one of the preceding claims characterised in that the container (11) is made from a liquid impermeable material.

5. An apparatus according to any one of the preceding claims characterised in that the receiving means (14) comprises a tube internally defining the hollow (19), the tube extending downwardly into the container (11) and having a sufficient length to support an article (20) placed in the hollow (19) with the article (20) temporarily supported by the closure member (22), the closure member (22) being at the bottom of the tube, and being hinged to the tube and movable by pivoting about a hinge axis (24) against the force of the resilient means (25).

6. An apparatus according to any one of the preceding characterised in that the resilient means (25) comprises a spring which is sufficiently strong to support the weight of an article (20) placed in the hollow (19) prior to operating of the operating means (27), and to return the closure member (22) to its closed position when the operating means (27) is released.

7. An apparatus according to any one of the preceding claims characterised in that the operating means (27) comprises a push rod which is attached to the closure member (22) and passes upwardly to a position adjacent the hollow (19) of the receiving means (14).

8. An apparatus according to any one of the preceding claims characterised in that the container (11) is generally cylindrical, the receiving means (14) being provided in an end face (L) of the cylinder, the hollow (19) of the receiving means (14) being spaced inwardly at least slightly from the side wall of the container (11).

9. An apparatus according to any one of the preceding claims characterised in that the container (11) comprises a body and a lid (L), the lid (L) being a snap fit with the body (B).

10. A method of disposing of a contaminated or dangerous article (20) using an apparatus (10) in accordance with any one of the preceding claims, characterised in that the method comprises placing the article (20) in the hollow (19) of the receiving means (14), operating the operating means (27) to cause the closure member (22) to move to the open position and allow the article (20) to pass into the container (11).

FIG 1

FIG 2

FIG 3